# EUROPEAN PATENT SPECIFICATION

(11) **EP 0 709 507 B1**
(45) Date of publication and mention of the grant of the patent: **08.05.1996**
(21) Application number: 94309133.0
(22) Date of filing: 07.12.1994
(51) Int. Cl.: D04H 1/64

(54) **Sanitiser in polymer and its use in non-wovens**
Entkeimer in einem Polymer und dessen Anwendung in Vliesstoffen
Assainisseur dans polymère et son utilisation dans les non-tissés

(43) Date of publication of application: 01.05.1996
(73) Proprietor: VINAMUL LTD., Carshalton, Surrey SM5 2JU (GB)
(72) Inventor: McLennan, Alistair John, Hackbridge Surrey SM6 7HL (GB); Kapadia, Surendra Nanalal, West Croydon Surrey CR0 3BU (GB)
(74) Representative: Wiesenhaan, Herman, Drs.

(56) References cited:
- EP-A- 0 113 254
- EP-A- 0 133 277
- EP-A- 0 147 759
- US-A- 4 590 102

## Description

This invention relates to certain polymeric compositions intended for use as a binding composition for fibrous materials such as viscose and polyester so as to obtain non-woven materials and to the non-woven materials obtained.

Certain non-woven materials such as wiping cloths ("wipes"), packaging materials, non-woven bandages etc. function often as short lived disposable articles because after some use they are prone to rapid biodeterioration. Moreover prolonged use of e.g. wiping cloths may lead to bad sanitary conditions e.g. due to undesired spread of bacteria and moulds. That is why such articles are usually discarded after having been used only once. Washing may help to extend the lifespan of these articles somewhat but is often found too cumbersome.

The present invention aims at providing certain non-woven articles such as wiping cloths etc. having a longer resistance to biodeterioration and a longer lifespan than is normally available, and the usage of such non-woven articles may help to improve sanitary conditions in hospitals, laboratories, catering establishments and households in general. Consequently "wipes" for industrial and domestic use are provided.

The present invention also provides certain polymeric compositions suitable as binding compositions for the non-woven articles according to the present invention.

European Patent Specification (EP-A-) 0 201 214 (Nippon Paint Co., Ltd) discloses polymeric microparticles containing pesticidal agents such as e.g. biocides against various organisms. These microparticles can be formulated in aqueous and non-aqueous formulations and serve for protecting various industrial products such as coating compositions, electric wire coverings, plastic products and the like, from being attacked by noxious animals and plants.

EP-A-0 147 759 (Goodrich Co B.F.) discloses an acrylic latex prepared by free radical polymerisation after which a few percent of an odour inhibition agent (anti-oxidant) is added to prevent degradation of the polymer backbone upon sterilisation. The acrylic polymer is hydrophobic and does not contain hydrophillic monomers such as ethylacrylate or vinylester. The polymer contains N-alkylol acrylamide as a cross-linking agent. The odour inhibition agent is a fully or partially hindered phenol carrying bulky groups like t.butyl in the ortho positions or an amine anti-oxidant. The odour inhibition agent is normally post-added (i.e. after polymerisation), and it is stated that this agent can also be added during polymerisation, although one would expect a disastrous effect of these anti-oxidants on the free radical polymerisation. The latex composition is used to bond fibres into non-wovens requiring repellency to body fluids and the non-wovens are used in medical/surgical applications without causing offensive odours upon sterilisation.

EP-A-0 113 254 (Tultex Corp.) discloses an anti-microbial non-woven product. Such non-woven fabrics are manufactured by forming a web of fibres and applying a binding agent such as an acrylic latex and the selected anti-microbial agent is added to the latex (post-dosed), becomes incorporated in colloidal suspension within the amorphous zones of the polymeric matrix and the binding agent is then ready for application to the textile web. It is preferred to use 0.05 to 1% by weight of the anti-microbial agent on the polymer base. Page 6, lines 21 to 23 mention curing of the non-woven after applying to the fabric. Several specific anti-microbial agents are disclosed in the above context. Page 4, lines 26-30 say: " the selected antimicrobial agent is added to the base resin, and the two are either melted together and mixed, or the agent is put into solution using a solvent which is compatible with the selected binder, then agent and the binder are mixed." A microbial agent introduced by post-dosing is cold- or hot-washed out giving no protection after washing.

EP-A-0 133 277 (Air Prod. & Chem) discloses non-woven products obtained by using as a binder an aqueous dispersion of a mixture of vinyl acetate-ethylene copolymer and a vinyl acetate-ethylene cross-linking monomer such as N-methylol(meth)acrylate. This binder is applied to the web of fibres and moderately heated to cure the binder to form a cross-linked interpolymer and of course before the binder is applied it is admixed with suitable catalyst for the cross-linking monomer e.g. HCl in the case of N-methylol acrylamide. The degree of cross-linking plays an important role for the desired properties of the polymer such as seal temperature and wet tensile strength. No sanitiser is disclosed.

US-A-4 590 102 (Rosamilia Peter L et al) discloses a cured non-woven substrate obtained by impregnating a web with a vinyl acetate/ethylene/N-methylolacryamide or vinyl chloride/ethylene/N-methylolacryamide copolymer emulsion with a low temperature acidic curing agent of low pkₐ such as e.g. oxalic acid, maleic acid or sodium bisulphate. The impregnated non-woven is then dried and subsequently cured at a low temperature. No sanitiser is disclosed.

In a first embodiment the present invention provides a polymeric binding composition for non-woven articles comprising vinylester and alkyl(metha)acrylate and/or ethylene and at least one post polymerisation crosslinkable monomer which is obtainable by incorporating 0.1 to 5% (w.w.) calculated on the amount of monomeric material of at least one sanitiser of the halogenated phenol type in an aqueous polymerisation mixture and subsequent emulsion/suspension polymerisation under the influence of free radicals.

In a preferred embodiment of the invention the sanitiser of the halogenated phenol type comprises a dicyclic chlorinated phenol.

In another embodiment of the invention provides a non-woven material comprising a polymeric binding composition with incorporated sanitiser as set out above.

Under a sanitiser is here to be understood a composition comprising a compound of the halogenated phenol type having a number of valuable properties such as microbicidal and fungicidal activity, a good human skin compatibility, stability and should be non-volatile and soluble in oil. Preferably a sanitiser is also of low toxicity to humans and has no adverse effect on the degradation activity in sewage plants. Suitable sanitisers according to this invention are certain halogenated phenol derivatives as set out below. Dicyclic chlorinated phenol derivatives are generally preferred. Particularly preferred is 2,2'-dihydroxy-5,5'-dichloro-diphenylmethane (Panacide tradename Coalite Chemicals U.K.). This latter compound has an especially favourable combination of properties. Other useful sanitisers are e.g. 2,4,4'-trichloro-2-hydroxydiphenylether (tradename Triclosan), available from Ciba Dyes and Chemicals; 4-chloro-3,5-xylenol (PCMX), available from Nipa Laboratories as Nipacide PX (Nipacide is a tradename); 5-chloro-2-hydroxy-diphenylmethane (Chlorophene), available from Nipa Laboratories as Nipacide BCP); 2,4-dichloro-3,5-dimethylphenol (DCMX), available from Nipa Laboratories as Nipacide DX; 4-chloro-3-methylphenol (PCMC), available from Nipa Laboratories as Nipacide PC, orthophenylphenol (OPP), and chlorinated derivatives thereof as e.g. available from Coalite Chemicals under the tradename Cotane. The invention covers the use of any of the above sanitisers. Panacide, Triclosan and Chlorophene are preferred sanitisers and Panacide is particularly preferred. Mixtures of more than one sanitiser can be used often with favourable results.
The sanitiser(s) is (are) present at a level of about 0.1 to 5% (w.w.) calculated on the amount of monomeric material, but the exact level may depend somewhat on the nature of the compound(s) used.

Non-woven fibrous material comprises a consolidated mass of fibres which may be laid down by mechanical, chemical, pneumatic, electrical or vacuum means and deposited in a desired shape, for example as webs, mats or sheets, or in a three dimensional form. The fibres may be natural or synthetic or a combination. In general the fibres to be used will be selected to suit the desired end use of the non-woven product. The fibres may be derived from wood, i.e. cellulose, or may be viscose, polyester, cotton, rayon, wool etc. The fibres may be deposited in a random manner or may be deposited or aligned along particular axes. The non-woven product may contain layers of fibres oriented in a cross-layered manner to provide a uniform strength across the products.
The good quality non-woven products according to the present invention comprise a binding composition to ensure that the product maintains its physical integrity. The binding composition is introduced during or after formation of the non-woven product. Part of the binding composition will coat the fibres, but another part will be concentrated at the positions where the fibres touch. Subsequent treatment, usually by the application of heat, dries and/or cures the binding composition and gives structural integrity to the product. The non-woven product may be impregnated with the binding composition by dipping or immersing in the composition to provided sufficient pick-up. There is no restriction on the means of application of the binding composition and this can also be e.g. soaking, spraying, rolling, brushing or applying a solid binding composition in the form of a powder, particles or fibres. The non-woven product comprising the binding composition in the form of a web can then be passed between a pair of optionally heated pressure rollers to ensure the impregnation is uniform and so as to control the amount of binding composition applied.

The polymeric binding composition used according to the present invention is particularly a copolymer emulsion obtained by polymerizing unsaturated monomers in an emulsion system and more in particular a polymeric composition substantially prepared from vinylester and alkyl(metha)-acrylate and/or ethylene and at least one post-polymerisation crosslinkable monomer. Such copolymer emulsions can be prepared by means of processes which can be performed at ambient temperature and at atmospheric or superatmospheric pressure, e.g. up to 12,000 kPa. Suitable post-polymerisation crosslinkable monomers comprise e.g. N-methylolacrylamide and/or an alkylester of N-methylolacrylamide e.g. N- (n-butoxymethyl)acrylamide, N-methylolacrylamide and/or an alkylester of N-methylolacrylamide are particularly preferred. The presence of a post-polymerisation crosslinkable monomer improves the strength of the copolymer solid and thereby provides structural integrity to the non-woven product. The amount of post-polymerisation monomer is usually in the range of 0.5 to 10% by weight of the total monomer content.
Preferably not more than 90% by weight, more preferably between 40 and 80% by weight of the monomer content is vinyl C1 to C4 alkanoate, with the preferred material being vinyl acetate.

The copolymer emulsion may include a C2 to C4 alkylene monomer, e.g. ethylene, typically in an amount from 5% to 40% by weight of total monomer. Another possible co-monomer is selected from the alkyl (C2 to C10) (meth)acrylate class e.g., butylacrylate or 2-ethyl hexylacrylate, which will preferably be present in an amount from about 10% to about 60% by weight of the total monomer.

Particularly preferred is a polymeric composition in which the polymeric composition comprises vinylacetate and alkylacrylate or a polymeric composition in which the alkylacrylate comprises butylacrylate. In another preferred embodiment of the polymeric composition the alkylacrylate comprises 2-ethylhexylacrylate.

A further class of functional co-monomer is carboxyl-containing monomers, e.g. acrylic acid, methacrylic acid, crotonic acid, itaconic acid etc. Hydroxyalkyl (meth) acrylate monomers, e.g. hydroxyethyl acrylate, may also be included. Typical levels are up to 5% by weight of total monomer. Other functional monomers e.g. sodium vinyl sulphonate and acrylamide may also be included usually in small percentages. Preferred are compositions in which the weight ratio of vinylacetate : alkylacrylate: crosslinkable monomer is from 30 - 80 : 20 - 70 : 0.5 - 10, also preferred are polymeric compositions in which the weight ratio of vinylacetate : ethylene : crosslinkable monomer is from 60 - 90 : 10 - 40 : 0.5 - 10. Preferably the mixture of polymers consists substantially of vinylacetate, ethylene or alkyl(meth)acrylate ester and a crosslinkable monomer. It is preferred to incorporate the sanitiser in one of the monomeric substances prior to polymerization, preferably in the vinyl ester.
To stabilise the latex one or more surfactants of the nonionic or anionic type may also be included. Such surfactants are well known in the art and are described at length in: "surface Active Agents and Detergents", Vol. II, by Schwartz, Perry & Birch, Interscience Publishers Inc. 1958. Protective colloids like e.g. polyvinyl alcohol may also be added.
Any suitable initiator can be used, in the case of thermal polymerization the use of ammonium- or alkali metal persulphate is convenient and in the case of redox initiation a persulphate, hydrogen peroxide or an organic peroxide is useful together with ascorbic acid or sodium metabisulphite as a reducing agent.

The copolymer emulsion can be prepared by using conventional techniques, which are well known in the art. Polymer synthesis (Vols I and II) by Sandler & Karo (Academic Press 1974) and Preparative Methods of Polymer Chemistry (Interscience 1968), provide more complete preparative details.

In a preferred embodiment of the invention the sanitiser is incorporated in a polymeric composition, which is prepared by emulsion- or suspension polymerization technique where the sanitiser is dissolved in monomeric material followed by polymerization under the influence of free radicals.

In another embodiment of the invention the sanitiser in a polymeric composition as material is in the form of an aqueous latex, which is obtained as the result of the polymerization reaction as such or after concentration thereof.

Another embodiment of the invention is the use of a sanitiser incorporated in a polymeric composition according to the present invention as a binding composition with incorporated sanitiser to bind and sanitise non-woven materials.

In another embodiment the invention provides non-woven material comprising a binding composition with incorporated sanitiser in which the amount of polymeric binder with incorporated sanitiser is from 10 to 45 % (w.w.) based on the weight of the fibres.

In another embodiment of the invention packaging material is provided which is coated with a polymeric binder with incorporated sanitiser according to the present invention.

Advantages of the non-woven materials according to the present invention is that they are less prone to biodeterioration and have a longer sanitary life due to slow release of the sanitiser, which even survives washing of the non-woven article and that the articles assist in good sanitary conditions in hospitals, laboratories and households.

The invention is illustrated by the following non-limiting examples. All parts, percentages and ratios are on a weight basis unless otherwise indicated.

### Example 1

To a polymerisation reactor were charged 0.547 parts of nonylphenol ethoxylate 35 EO (Ethylan HA, tradename ex Akcros U.K.), 0.418 parts sodium salt of n. dodecylbenzenesulphonic acid (Arylan SC15, trademark ex Akcros U.K.) (15% solution), 0.111 parts of sodium acetate and 61.0 parts of deionised water. The reactor was heated to 70°C and purged with nitrogen.

In a separate vessel, 1.172 parts of Ethylan HA, 3.216 parts of Arylan SC15, (15% solution), and 7.540 parts of a 45% aqueous solution of N-methylolacrylamide were dissolved in 31.984 parts of deionised water to form a second water phase. In a separate vessel, 2.070 parts of 2,2'-dihydroxy-5,5'-dichloro-diphenylmethane (Panacide Technical Solid, ex Coalite U.K.) was dissolved in a monomer mixture comprising 34.735 parts of vinyl acetate and 42.382 parts of butyl acrylate. The solution of Panacide in monomers and the second water phase were mixed under agitation to produce a pre-emulsion.

When the reactor temperature was stable at 70°C, a mixture of 9.965 parts of vinyl acetate and 1.124 parts of butyl acrylate was charged, followed by a solution of 0.203 parts of ammonium persulphate in 1.608 parts of deionised water. The reactor contents were allowed to exotherm to 75°C, when the slow-addition of the pre-emulsion was commenced to last for 3½ hours. At the end of the pre-emulsion addition, a mixture of 3.766 parts of vinyl acetate and 4.635 parts of butyl acrylate was slow-added over 30 minutes. 30 minutes after the commencement of the pre-emulsion addition, a slow-addition of 0.111 parts of ammonium persulphate in 16.548 parts of deionised water was commenced to take 4 hours. The temperature was maintained at 75°C for the duration of the slow additions.

After the slow additions were complete, a solution of 0.055 parts of ammonium persulphate in 0.500 parts of deionised water was added, and the temperature was increased to 80°C for 1 hour. At the end of this period the temperature was reduced to 50°C, and solutions of 0.300 parts of t-butyl hydroperoxide in 1.000 part of deionised water, and 0.200 parts of sodium formaldehydesulphoxylate in 1.000 parts of deionised water were added over 30 minutes. A solution of 0.092 parts of an antifoaming agent (blend of mineral oil and silicon oil) in 1.000 parts of deionised water was added. The reactor was cooled and the product was discharged and filtered through nylon mesh to remove coagulum. The resultant latex had a solids content of 47.14%, pH of 3.7 and viscosity of 0.61 Pa.s.

### Comparative Example 1

To a polymerisation reactor was charged 0.536 parts of Ethylan HA, 0.409 parts of Arylan SC15, (15% solution), 0.109 parts of sodium acetate and 59.83 parts of deionised water. The reactor was heated to 70°C and purged with nitrogen.

In a separate vessel, 1.148 parts of Ethylan HA, 3.151 parts of Arylan SC15, (15% solution), and 7.388 parts of a 45% aqueous solution of N-methylolacrylamide were dissolved in 31.334 parts of deionised water to form a second water phase. In a separate vessel 38.734 parts of vinyl acetate and 47.078 parts of butyl acrylate were mixed. The monomer mixture and the second water phase were mixed under agitation to produce a pre-emulsion.

When the reactor temperature was stable at 70°C, a mixture of 9.763 parts of vinyl acetate and 1.101 parts of butyl acrylate was charged, followed by a solution of 0.199 parts of ammonium persulphate in 1.575 parts of deionised water. The reactor contents were allowed to exotherm to 75°C, when the slow-addition of the pre-emulsion was commenced to last for 4 hours. 30 minutes after the commencement of the pre-emulsion addition, a slow-addition of 0.109 parts of ammonium persulphate in 16.213 parts of deionised water was commenced to take 4 hours. The temperature was maintained at 75°C for the duration of the slow additions.

After the slow additions were complete, the temperature was increased to 80°C for 1 hour. At the end of this period the temperature was reduced to 50°C and a solution of 0.091 parts of an antifoaming agent (equal parts of mineral oil and silicon oil) in 1.630 parts of deionised water was added. The reactor was cooled and the product was discharged and filtered through nylon mesh to remove coagulum. The resultant latex had a solids content of 45.81%, pH of 4.25 and viscosity of 0.35 Pa.s.

### Example 2

The binders of Example 1 and Comparative Example 1 were diluted with water to 20% solids, catalysed by the addition of 1% ammonium chloride w/w, and used to impregnate a light viscose web of weight 15 g/m, giving a pick-up of ca. 19.5%. The webs were dried and cured at 165°C on a rotary iron for 12 seconds. Strips of impregnated web of dimensions 200 x 50 mm were then tested for tensile strength on an Instron Tensiometer. The breaking load in kg and elongation at break in % was measured for dry samples, wet samples and samples dipped in solvent, (acetone).

| | Example 1 | Comparative Example 1 |
|---|---|---|
| Dry Tensile Strength, kg | 0.704 | 0.723 |
| Elongation, % | 26.03 | 23.94 |
| Wet Tensile Strength, kg | 0.265 | 0.254 |
| Elongation, % | 32.68 | 32.87 |
| Solvent Tensile Strength, kg | 0.118 | 0.145 |
| Elongation, % | 7.82 | 8.56 |

This shows that the Panacide does not adversely affect the performance as a non-woven binder.

### Example 3

Non-woven fabrics were impregnated with binder formulations as detailed:-

| | A | B | C |
|---|---|---|---|
| Latex of Example 1 | 260 | - | - |
| Latex of Comparative Example 1 | - | 257.7 | 260 |
| Wetting Agent* | 2.1 | 2.1 | 2.1 |
| Citric Acid | 0.8 | 0.8 | 0.8 |
| Water | 337.1 | 327.9 | 337.1 |
| Panacide Solid | - | 2.3 | - |
| Propylene Glycol | - | 9.2 | - |

| | | | |
|---|---|---|---|
| * = sodium salt of dioctyl sulphosuccinate (70% active) | | | |

Fabrics were cured, then some were washed cold at 30°C and some were washed hot at 70°C. Discs of each fabric were cut out and placed on to specific agar media. The media used were Nutrient Agar for bacteria and Rose Bengal, Chloramphenicol Agar and Malt Extract Agar for fungi and yeasts. The discs of fabric were then inoculated with an appropriate spore suspension inoculum, which contained ca. 10⁷ spores per ml. After incubation for 7 days at 30°C for bacteria and 27-28°C for fungi and yeasts the plates were examined and the amount of growth noted.

| | No Wash | Cold Wash | Hot Wash |
|---|---|---|---|
| A | No Growth | No Growth | No Growth |
| B | Heavy Growth | Heavy Growth | Heavy Growth |
| C | Heavy Growth | Heavy Growth | Heavy Growth |

This demonstrates that the inclusion of Panacide by encapsulation in a non-woven binder protects fabric impregnated with the binder, whereas fabric impregnated with binder which does not contain Panacide, or contains only post-added Panacide does not protect the fabric impregnated with the binder. Similar results were obtainable with Triclosan and Chlorophene.

## Claims

1. A polymeric binding composition for non-woven articles comprising vinylester and alkyl(metha)acrylate and/or ethylene and at least one post polymerisation crosslinkable monomer which is obtainable by incorporating 0.1 to 5% (w.w.) calculated on the amount of monomeric material of at least one sanitiser of the halogenated phenol type in an aqueous polymerization mixture and subsequent emulsion/suspension polymerisation under the influence of free radicals.

2. A polymeric binding composition according to claim 1 in which the sanitiser of the halogenated phenol type comprises a dicyclic chlorinated phenol.

3. A polymeric binding composition according to claim 1 or 2 which comprises vinylacetate and alkylacrylate.

4. A polymeric binding composition according to claim 1, 2 or 3, in which the alkylacrylate comprises butylacrylate.

5. A polymeric binding composition according to claim 1, 2 or 3, in which the alkylacrylate comprises 2-ethylhexylacrylate.

6. A polymeric binding composition as claimed in any of the claims 1 to 5, in which the crosslinkable monomer comprises N-methylolacrylamide and/or an alkylester of N-methylolacrylamide.

7. A polymeric binding composition as claimed in any of the claims 1 to 6, in which the weight ratio of vinylacetate : alkylacrylate: crosslinkable monomer is from 30 - 80 : 20 - 70 : 0.5 - 10.

8. A polymeric binding composition as claimed in claim 7, in which the weight ratio of vinylacetate : ethylene : crosslinkable monomer is from 60 - 90 : 10 - 40 : 0.5 - 10.

9. A polymeric binding composition as claimed in any of the claims 1 to 8, in which the sanitiser is selected from the group consisting of 2,2'-dihydroxy-5,5'dichlorodiphenyllmethane, 2,4,4'-trichloro-2-hydroxydiphenylether and 5-chloro-2-hydroxy-diphenylmethane.

10. A polymeric binding composition as claimed in any of the claims 1 to 9, in which the sanitiser comprises 2,2'-dihydroxy-5,5'-dichloro-diphenylmethane.

11. A polymeric binding composition as claimed in any of the claims 1 to 10, in which the polymeric material is present in the form of an aqueous latex.

12. A process for preparing a polymeric binding composition for non-woven articles by emulsion- or suspension polymerization under the influence of free radicals of an aqueous reaction mixture comprising vinylester and alkyl(metha)acrylate and/or ethylene and at least one post polymerisation crosslinkable monomer in the presence of 0.1 to 5% (w.w.) calculated on the amount of monomeric material of at least one sanitiser of the halogenated phenol type.

13. Use of a polymeric binding composition with incorporated sanitiser as claimed in any of the claims 1 to 11, in which the composition is used to bind and sanitise non-woven materials.

14. A non-woven material comprising a polymeric binding composition with incorporated sanitiser according to any of the claims 1-11.

15. A non-woven material according to claim 14 in which the amount of polymeric binding composition with incorporated sanitiser is from 10 to 45 % (w.w.) based on the weight of the fibres.

16. Packaging material coated with a polymeric binding composition with incorporated sanitiser according to any of the claims 1-11.

## Patentansprüche

1. Poylmere Bindemittelzusammensetzung für Nonwoven-Artikel, umfassend Vinylester und Alkyl(meth)acrylat und/oder Ethylen und mindestens ein nach der Polymerisation vernetzbares Monomer, das unter Einverleibung von 0,1 bis 5% (Gew./Gew.), berechnet auf die Menge an Monomermaterial, mindestens eines Entkeimers des halogenierten Phenoltyps in ein wäßriges Polymerisationsgemisch und nachfolgende Emulsions-/Suspensions-Polymerisation unter dem Einfluß von Radikalen erhältlich ist.

2. Polymere Bindemittelzusammensetzung nach Anspruch 1, in der der Entkeimer des halogenierten Phenoltyps ein bicyclisches chloriertes Phenol umfaßt.

3. Polymere Bindemittelzusammensetzung nach Anspruch 1 oder 2, die Vinylacetat und Alkylacrylat umfaßt.

4. Polymere Bindemittelzusammensetzung nach Anspruch 1, 2 oder 3, in der das Alkylacrylat Butylacrylat umfaßt.

5. Polymere Bindemittelzusammensetzung nach Anspruch 1, 2 oder 3, in der das Alkylacrylat 2-Ethylhexylacrylat umfaßt.

6. Polymere Bindemittelzusammensetzung nach einem der Ansprüche 1 bis 5, in der das vernetzbare Monomer N-Methylolacrylamid und/oder einen Alkylester von N-Methylolacrylamid umfaßt.

7. Polymere Bindemittelzusammensetzung nach einem der Ansprüche 1 bis 6, in der das Gewichtsverhältnis von Vinylacecat : Alkylacrylat : vernetzbarem Monomer 30-80:20-70:0,5-10 beträgt.

8. Polymere Bindemittelzusammensetzung nach Anspruch 7, in der das Gewichtsverhältnis von Vinylacetat : Ethylen : vernetzbarem Monomer 60-90:10-40:0,5-10 beträgt.

9. Polymere Bindemittelzusammensetzung nach einem der Ansprüche 1 bis 8, in der der Entkeimer aus der aus 2,2'-Dihydroxy-5,5'-dichlordiphenylmethan, 2,4,4'-Trichlor-2-hydroxydiphenylether und 5-Chlor-2-hydroxy-diphenylmethan bestehenden Gruppe ausgewählt ist.

10. Polymere Bindemittelzusammensetzung nach einem der Ansprüche 1 bis 9, in der der Entkeimer 2,2'-Dihydroxy-5,5'-dichlordiphenylmethan umfaßt.

11. Polymere Bindemittelzusammensetzung nach einem der Ansprüche 1 bis 10, in der das polymere Material in Form eines wäßrigen Latex vorliegt.

12. Verfahren zur Herstellung einer polymeren Bindemittelzusammensetzung für Nonwoven-Artikel durch Emulsions- oder Suspensionspolymerisation unter dem Einfluß von Radikalen eines wäßrigen Reaktionsgemisches, welches Vinylester und Alkyl(meth)acrylat und/oder Ethylen und mindestens ein nach der Polymerisation vernetzbares Monomer umfaßt, in Gegenwart von 0,1 bis 5% (Gew./Gew.), berechnet auf die Menge an Monomermaterial, mindestens eines Entkeimers des halogenierten Phenoltyps.

13. Verwendung einer polymeren Bindemittelzusammensetzung mit einverleibtem Entkeimer nach einem der Ansprüche 1 bis 11, wobei die Zusammensetzung zum Binden und Keimfreimachen von Nonwoven-Materialien verwendet wird.

14. Nonwoven-Material, umfassend eine polymere Bindemittelzusammensetzung mit einverleibtem Entkeimer nach einem der Ansprüche 1 bis 11.

15. Nonwoven-Material nach Anspruch 14, in dem die Menge an polymerer Bindemittelzusammensetzung mit einverleibtem Entkeimer 10 bis 45% (Gew./Gew.), bezogen auf das Fasergewicht, beträgt.

16. verpackungsmaterial, beschichtet mit einer polymeren Bindemittelzusammensetzung mit einverleibtem Entkeimer nach einem der Ansprüche 1 bis 11.

## Revendications

1. Composition liante polymère pour des articles non tissés comprenant un ester vinylique et un (méth)acrylate d'alkyle et/ou de l'éthylène et au moins un monomère réticulable de post-polymérisation que l'on peut obtenir en incorporant 0,1 à 5% (p/p) calculé sur la quantité de matière monomère d'au moins un assainisseur du type phénol halogéné dans un mélange de polymérisation en phase aqueuse et une polymérisation en émulsion/suspension ultérieure sous l'influence de radicaux libres.

2. Composition liante polymère selon la revendication 1, dans laquelle l'assainisseur du type phénol halogéné comprend un phénol chloré dicyclique.

3. Composition liante polymère selon la revendication 1 ou 2, qui comprend de l'acétate de vinyle et de l'acrylate d'alkyle.

4. Composition liante polymère selon la revendication 1, 2 ou 3, dans laquelle l'acrylate d'alkyle comprend l'acrylate de butyle.

5. Composition liante polymère selon la revendication 1, 2 ou 3, dans laquelle l'acrylate d'alkyle comprend l'acrylate de 2-éthylhexyle.

6. Composition liante polymère selon l'une quelconque des revendications 1 à 5, dans laquelle le monomère réticulable comprend le N-méthylolacrylamide et/ou un ester alkylique de N-méthylolacrylamide.

7. Composition liante polymère selon l'une quelconque des revendications 1 à 6, dans laquelle le rapport pondéral d'acétate de vinyle:acrylate d'alkyle:monomère réticulable est de 30 à 80:20 à 70:0,5 à 10.

8. Composition liante polymère selon la revendication 7, dans laquelle le rapport pondéral d'acétate de vinyle:éthylène:monomère réticulable est de 60 à 90:10 à 40:0,5 à 10.

9. Composition liante polymère selon l'une quelconque des revendications 1 à 8, dans laquelle l'assainisseur est choisi dans le groupe formé par le 2,2'-dihydroxy-5,5'-dichlorodiphénylméthane, le 2,4,4'-trichloro-2-hydroxydiphényléther et le 5-chloro-2-hydroxy-diphénylméthane.

10. Composition liante polymère selon l'une quelconque des revendications 1 à 9, dans laquelle l'assainisseur comprend le 2,2'-dihydroxy-5,5'-dichloro-diphénylméthane.

11. Composition liante polymère selon l'une quelconque des revendications 1 à 10, dans laquelle la matière polymère est présente sous forme d'un latex aqueux.

12. Procédé pour préparer une composition liante polymère pour des articles non tissés par polymérisation en émulsion ou en suspension sous l'influence de radicaux libres d'un mélange réactionnel aqueux comprenant de l'ester de vinyle et du (métha)acrylate d'alkyle et/ou de l'éthylène et au moins un monomère réticulable de post-polymérisation en présence de 0,1 à 5% (p/p) calculé sur la quantité de matière monomère d'au moins un assainisseur du type de phénol halogéné.

13. Utilisation d'une composition liante polymère avec un assainisseur incorporé selon l'une quelconque des revendications 1 à 11, dans laquelle on utilise la composition pour lier et assainir les matières non tissées.

14. Matière non tissée comprenant une composition liante polymère avec un assainisseur incorporé selon l'une quelconque des revendications 1 à 11.

15. Matière non tissée selon la revendication 14, dans laquelle la quantité de composition liante polymère avec un assainisseur incorporé est comprise entre 10 et 45% (p/p) par rapport au poids des fibres.

16. Matière d'emballage revêtue d'une composition liante polymère avec un assainisseur incorporé selon l'une quelconque des revendications 1 à 11.
